(19) Europäisches Patentamt
European Patent Office
Office européen des brevets

(11) Publication number: **0 055 997**
**B1**

(12) **EUROPEAN PATENT SPECIFICATION**

(45) Date of publication of patent specification: **26.02.86**

(51) Int. Cl.[4]: **C 07 D 303/08,** C 07 C 33/46
// C07D249/08, A01N43/64

(21) Application number: **82200174.9**

(22) Date of filing: **06.03.80**

(60) Publication number of the earlier application in accordance with Art. 76 EPC: **0 015 756**

(54) **Oxirane and halohydrin compounds.**

(30) Priority: **07.03.79 GB 7908003**
**21.09.79 GB 7932819**

(43) Date of publication of application:
**14.07.82 Bulletin 82/28**

(45) Publication of the grant of the patent:
**26.02.86 Bulletin 86/09**

(84) Designated Contracting States:
**BE CH DE FR GB IT NL**

(56) References cited:
**FR-A-1 408 958**
**GB-A- 932 487**
**GB-A-1 448 437**
**GB-A-1 532 156**
**US-A-3 800 044**

**EUROPEAN JOURNAL OF MEDICINAL CHEMISTRY, CHIMICA THERAPEUTICA, vol. XIV, no. 2, March-April, 1979, PARIS (FR), H. GALONS et al.: "Synthèse et étude pharmacologique de phényl-3 propanol-2 amines", pages 165-170**

(73) Proprietor: **IMPERIAL CHEMICAL INDUSTRIES PLC**
**Imperial Chemical House Millbank**
**London SW1P 3JF (GB)**

(72) Inventor: **Parry, Keith Peter**
**7 Juniper Drive Off Ray Park Road**
**Maidenhead Berkshire (GB)**
Inventor: **Worthington, Paul Anthony**
**22, Boulters Gardens**
**Maidenhead Berkshire (GB)**
Inventor: **Rathmell, William George**
**'Culvers' 10, Gypsy Lane**
**Wokingham Berkshire (GB)**

(74) Representative: **Alner, Henry Giveen Hamilton et al**
**Imperial Chemical Industries PLC Legal Department: Patents P.O. Box 6 6, Bessemer Road**
**Welwyn Garden City Herts, AL7 1HD (GB)**

**EP 0 055 997 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to oxirane and halohydrin compounds which can be used to prepare triazole compounds having fungicidal activity. Such fungicidal compounds are described and claimed in European Patent No. 15756

The invention provides compounds of the general formula

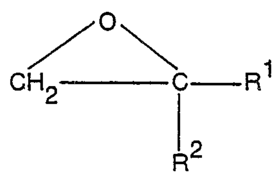
and
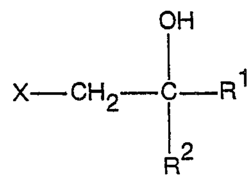

wherein X is a halogen atom, $R^1$ is butyl (especially *t*-butyl), 2-chloro- or 2-fluorophenyl, or cycloakyl and $R^2$ is 2-chlorobenzyl but only when $R^1$ is butyl or cycloalkyl, or $R^2$ is:

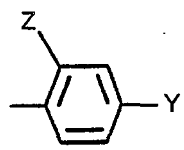

wherein Z is hydrogen or chlorine and Y is chlorine or fluorine provided that Z and Y are both chlorine only when $R^1$ is butyl.

More specifically the present invention provides compounds as defined above wherein $R^1$ is butyl (*n-, sec-, iso-* or *t*-butyl), 2-chloro- or 2-fluorophenyl, or cycloalkyl and $R^2$ is 2-chlorobenzyl, 4-chlorophenyl, or 4-fluorophenyl or 2-chloro-3-fluorophenyl or 2,4-dichlorophenyl only when $R^1$ is butyl. In particular the invention provides chemical compounds as defined above wherein $R^1$ is *t*-butyl, 2-chlorophenyl or 2-fluorophenyl and $R^2$ is 4-flourophenyl or 4-chlorophenyl.

Specific oxirane compounds according to the invention are of formula

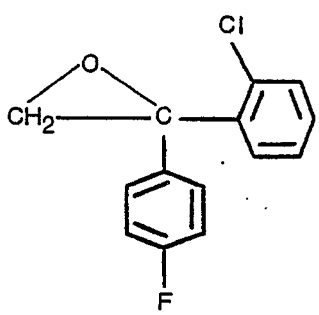
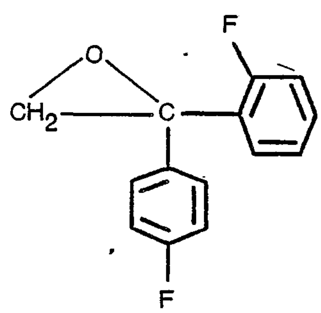
and
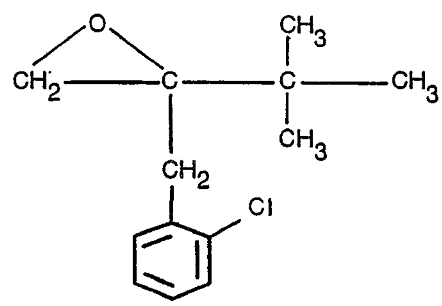

Specific halohydrin compounds according to the invention are compounds having the specific formuale below wherein X is chlorine or bromine:

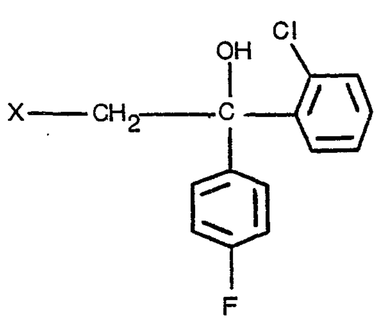
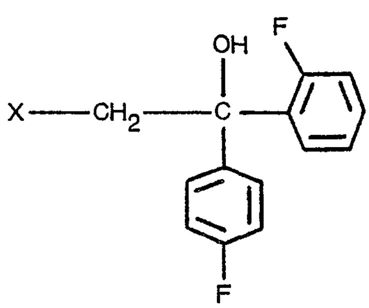
and
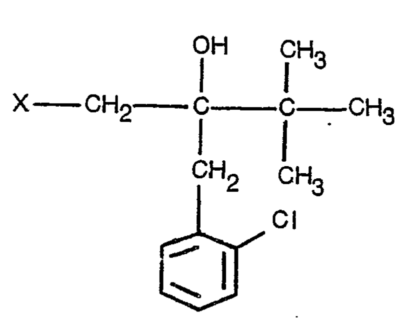

Examples of triazole compounds useful as fungicides which may be prepared using the intermediate oxirane and halohydrin compounds of the present invention are shown in Table I below.

The triazole compounds have the general formula:

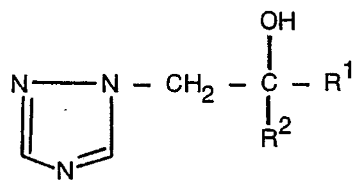

TABLE I

| COMPOUND NO | $R^1$ | $R^2$ | MELTING POINT (°C) |
|---|---|---|---|
| 1 | $C_6H_5$— | $C_6H_5CH_2$— | 124—125 |
| 2 | $C_6H_5$— | p-Cl—$C_6H_4CH_2$— | 144—145 |
| 3 | $C_6H_5$— | p-F—$C_6H_4CH_2$— | 166—118 |
| 4 | p-Cl—$C_6H_4$— | p-Cl—$C_6H_4CH_2$— | 80—83 |
| 5 | p-Cl—$C_6H_4$— | $C_6H_5CH_2$— | 109—11 |
| 6 | p-F—$C_6H_4$— | $C_6H_5CH_2$— | 141—142 |
| 7* | $C_6H_5$— | 2,4-diCl—$C_6H_3CH_2$— | 104—106 |
| 8+ | p-F—$C_6H_4$— | p-F—$C_6H_4CH_2$— | 154—156 |
| 9 | p-F—$C_6H_4$— | p-Cl—$C_6H_4CH_2$— | 168—170 |
| 10 | t-Bu | $C_6H_5CH_2$— | 110—110 |
| 11 | t-Bu | p-Cl—$C_6H_4CH_2$ | 86—87 |
| 12 | t-Bu | p-F—$C_6H_4CH_2$— | 146—148 |
| 13 | $C_6H_5$— | o-F—$C_6H_4CH_2$— | 133—134 |
| 14 | p-Cl—$C_6H_4$— | o-F—$C_6H_4CH_2$— | 95—96 |
| 15 | $C_6H_5$— | o-Cl—$C_6H_4CH_2$— | 69—71 |
| 16 | p-MeO—$C_6H_4$— | $C_6H_5CH_2$— | 100—103 |
| 17 | $C_6H_5$— | $C_6H_5$— | 128—129 |
| 18+ | p-F—$C_6H_4$— | p-F—$C_6H_4CH_2$— | 161—163 |
| 19 | $C_6H_5$— | 2,4-diCl—$C_6H_3CH_2$— | 104—106 |
| 20 | t-Bu | o-Cl—$C_6H_4CH_2$— | 74—75 |
| 21 | t-Bu | m-F—$C_6H_4CH_2$— | 96—98 |
| 22 | t-Bu | m-Cl—$C_6H_4CH_2$— | 88—89 |
| 23 | t-Bu | m-$CF_3$—$C_6H_4CH_2$— | 106—107 |
| 24 | $C_6H_5$— | p-t-Bu-$C_6H_4CH_2$— | 80—83 |
| 25 | p-Cl—$C_6H_4$— | $C_6H_5$— | 83—85 |
| 26 | p-Cl—$C_6H_4$— | p-Cl—$C_6H_4$— | 147—148 |

| Compound No. | $R^1$ | $R^2$ | Melting Point (°C) |
|---|---|---|---|
| 27 | p-Cl—$C_6H_4$— | p-F—$C_6H_4$— | 154—155 |
| 28 | 2,4-diCl—$C_6H_3$— | $C_6H_5$— | 191—194 |
| 29 | p-F—$C_6H_4$— | p-F—$C_6H_4$— | 170—171 |
| 31 | i-Bu | $C_6H_5$— | 94—95 |
| 32 | n-Bu | p-Cl—$C_6H_5$— | 95—97 |
| 33 | t-Bu | 2-Cl-6-F-$C_6H_3CH_2$— | |
| 34 | t-Bu | 2-Cl-4-F—$C_6H_3CH_2$— | |
| 35 | t-Bu | 2-F-4-Cl—$C_6H_3CH_2$— | |
| 36 | t-Bu | 2,4-diCl—$C_6H_3CH_2$— | |
| 37 | t-Bu | 2,6-diCl—$C_6H_3CH_2$— | |
| 38 | t-Bu | 2,6-diF—$C_6H_3CH_2$— | |
| 40 | $C_6H_5$— | p-t-Bu—$C_6H_4$ | |
| 41 | $C_6H_5$— | o-Cl—$C_6H_4$— | |
| 42 | $C_6H_5$— | o-F—$C_6H_4$— | |
| 43 | p-Cl—$C_6H_4$— | o-Cl—$C_6H_4$ | 137—138 |
| 44 | p-Cl—$C_6H_4$— | o-F—$C_6H_4$— | 144—145 |
| 45 | p-F—$C_6H_4$— | o-Cl—$C_6H_4$— | 115—116 |
| 46 | p-F—$C_6H_4$— | o-F—$C_6H_4$— | 120—123 |
| 47 | $C_6H_5$— | o-$C_6H_5$—$C_6H_4$— | |
| 48 | p-Cl—$C_6H_4$— | o-$C_6H_5$—$C_6H_4$— | |
| 49 | $C_6H_5$— | o-$C_6H_5O$—$C_6H_4$— | |
| 50 | p-Cl—$C_6H_4$— | o-$C_6H_6O$—$C_6H_4$— | |
| 51 | $C_6H_5$— | o-Me—$C_6H_4$— | |
| 52 | p-Cl—$C_6$H4— | o-Me—$C_6H_4$— | |
| 53 | 2,4-diCl—$C_6H_3$— | p-F—$C_6H_4$— | 137—138 |

* Includes 1 mole of ethanol occluded in the crystal lattice

+ Compounds 8 and 19 were obtained as polymorphs and this explains their different melting points

The compounds of Table I may be produced by reacting a compound of the general formula

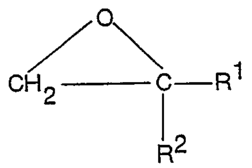

or

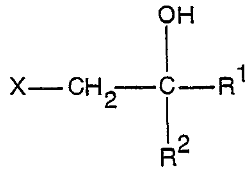

in which $R^1$ and $R^2$ are as defined above and X is a halogen atom (preferably a chlorine or bromine atom), with 1,2,4-triazole either in the presence of an acid-binding agent or in the form of one of its alkali metal salts in a convenient solvent. Suitably the oxirane or halohydrin compounds of the general formulae above are reacted at 20—100°C with the sodium salt of 1,2,4-triazole (the salt can be prepared by adding either sodium hydride or sodium methoxide to 1,2,4-triazole) in a convenient solvent such as acetonitrile, methanol, ethanol or dimethylformamide. The product can be isolated by pouring the reaction mixture into water and recrystallising the solid formed from a convenient solvent.

The oxirane and halohydrin compounds of the present invention can be prepared by reacting a compound of general formula

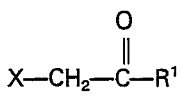

or

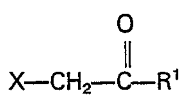

wherein $R^1$, $R^2$ and X are as defined above with, respectively, a Grignard comound of general formula

$$Y—Mg—R^2 \text{ or } Y—Mg—R^1$$

wherein $R^1$ and $R^2$ are as defined above and Y is a halogen (preferably chlorine, bromine or iodine) in a convenient solvent such as diethyl ether or tetrahydrofuran. Generally a mixture of the oxirane and halohydrin compounds of the general formulae shown are obtained. For example, when a ketone compound of the first (left-hand side) general formula wherein $R^1$ is alkyl or cycloalkyl is reacted, the oxirane compound generally predominates in the mixture; on the other hand, when $R^1$ is optionally substituted phenyl, the halohydrin compound generally predominates in the mixture.

The ketone compounds of the general formula shown above may be made by methods set out in the literature.

The oxirone compounds of the above general formulae wherein each of $R^1$ and $R^2$, which may be the same or different, is substituted phenyl may also be prepared by reacting the appropriate benzophenone compound of general formula

$$R^1—CO—R^2$$

wherein $R^1$ and $R^2$ are as defined above, with dimethyl oxosulphonium methylide (Corey and Chaykovsky, JACS, 1965, *87*, 1353—1364) or dimethyl sulphonium methylide (Corey and Chaykovsky, JACS, 1962, *84*, 3782) using methods set out in the literature.

The benzophenone compounds of the general formula shown can be prepared, using the Friedel-Crafts reaction, by reacting a substituted benzoyl chloride with the appropriately substituted benzene in the presence of a Lewis acid, e.g. aluminium chloride.

The oxirane compounds of the invention wherein each of $R^1$ and $R^2$ are as defined above can also be produced by reacting a β-hydroxy selenide compound of general formula

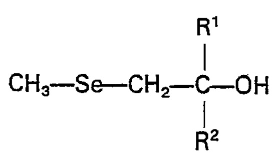

wherein $R^1$ and $R^2$ are as defined above, with methyl iodide in potassium t-butoxide according to the method of Van Ende, Dumont and Krief, Angew. Chem. Int. Ed., 1975 *14,* 700.

The β-hydroxy selenide compound can be prepared by treating the diselenide with the appropriate ketone in the presence of butyl lithium.

The triazole compounds listed in Table I are fungicidally active, particularly against the diseases : *Piricularia oryzae* on rice, *Puccinia recondiat, Puccinia striiformis* and other rusts on wheat, *Puccania hordie, Puccinia strifformis* and other rusts on barley, and rusts on other hosts, eg. coffee, apples,

vegetables and ornamental plants, *Plasmopara viticola* on vines, *Erysiphe graminis* (powdery mildew) on barley and wheat and other powdery mildews on various hosts such as *Sphareotheca fuliginea* on cucurbits (e.g. cucumber), *Podosphaera leucotricha* on apples and Uncinula necator on vines, *Helminthosporium* spp. and *Rhynchosporium* spp. on cereals, *Cercospora arachidicola* on penuts and other *Cercospora* species on for example sugar beet, bananas and soya beans, *Botrytis cinerea* (grey mould) on tomatoes, strawberries, vines and other hosts, *Phytophthora infestans* (late blight) on tomatoes, *Venturia inaequalis* (scab) on apples.

Some of the compounds have also shown a broad range of activities against fungi *in vitro.* They have activity against various post-harvest diseases on fruit (eg. *Penicillium digatatum* and *italicum* on oranges and *Gloeosporium musarum* on bananas). Further some of the compounds are active as seed dressings against: *Fusarium* spp., *Septoria* spp., *Tilletia* spp. (ie. bunt, a seed borne disease of wheat), *Ustilago* spp., *Helminthosporium* spp. on cerals, *Rhizoctonia solani* on cotton and *Corticium sasakii* on rice.

The compounds can move acropetally in the plant tissue. Moreover, the compounds can be volatile enough to be active in the vapour phase against fungi on the plant.

The compounds may be used as such for fungicidal purposes but are more conveniently formulated into compositions for such usage.

The following Example 5 illustrates the invention; the temperatures are given in degrees Centrigrade (°C). The Example 5 describes the preparation of a compound according to the invention, and its use to prepare triazole fungicides listed in Table I. The chemical name of this fungicide is set out at the beginning of each Example. Examples 1—4 describe the preparation of compounds analogous to those according to the invention and their use to prepare triazole fungicides listed in Table I.

Example 1

1-(1,2,4-Triazol-1-yl)-2,3-diphenyl-propan-2-ol (Compound No. 1)

Benzyl chloride (0.2 mol) was dissolved in dry diethyl ether (200 ml) and added dropwise to magneisum turnings (0.22 g atoms). After all the magnesium had reacted, the solution was refluxed for 1 hour and cooled to room temperature. Phenacyl chloride (0.1 mol) in dry diethyl ether (100 ml) was added dropwise over 1 hour at such a rate as to maintain gentle reflux. The solution was then refluxed for 2 hours, and cooled to room temperature; the mixture was poured into ice and the complex decomposed with ammonium chloride solution. The etheral solution was washed several times with water (2 × 200 ml), dried ($Na_2SO_4$), and the solvent removed *in vacuo* to give, as a colourless oil, the crude chlorohydrin which was dissolved in dimethyl formamide (80 ml) and a solution of sodium triazole [prepared from sodium (0.1 g atoms) in methanol (40 ml) and 1,2,4-triazole (0.1 mol)] added dropwise at room temperature. After stirring at room temperature for 2 hours, the solution was warmed at 50°C for 3 hours. The solvent was removed *in vacuo* and the residue poured into water to give a crystalline solid which was recrystallised from ethanol/petroleum ether to give the title compound, mp. 124.5°.

Example 2

1-(1,2,4-Triazol-yl)-2-phenyl-3-p-fluorophenyl-propan-2-ol (Compound 3)

p-Fluorobenzyl chloride (0.1 mol) in dry diethyl ether (100 ml) was added dropwise to magnesium turnings (0.11 g atoms) and the solution stirred vigorously until refluxing occurred. When all the magnesium had reacted, the solution was refluxed for a further 1 hour and then cooled to room temperature. Phenacyl chloride (0.05 ml) in dry diethyl ether (50 ml) was added dropwise to the solution over 1 hour at such a rate as to maintain gentle reflux. The mixture was refluxed for 2 hours, cooled to room temperature and the mixture poured into ice/ammonium chloride solution to decompose the complex. The ethereal solution was washed several times with water (2 × 200 ml), dried ($Na_2SO_4$), and the solvent removed *in vacuo* to give, as a colourless oil, the crude chlorohydrin. The latter was dissolved in dimethylformamide (40 ml) and a solution of sodium triazole [prepared from sodium (0.05 g atoms) in methanol (20 ml) and 1,2,4-triazole (0.05 mol)] added dropwise at room temperature. After stirring at room temperature for 2 hours, the solution was warmed at 50° for 3 hours. The solvent was removed *in vacuo* and the mixture poured into water to give a crystalline solid which was recrystallised from petroleum ether/chloroform to give the title compound, mp. 116—8°.

Example 3

1-(1,2,4-triazol-1-yl)-2,2-diphenyl-ethan-2-ol (Compound 17)

*Stage 1.* Bromobenzene (0.2 mol, 31.4 g) on sodium dry diethyl ether (200 ml) was added dropwise to magnesium (0.22 gram atoms, 5.3 g). After all the magnesium had reacted, phenacyl chloride (0.1 mol, 15.5 g) in diethyl ether (100 ml) was added dropwise and the solution stirred at room temperature for 1 hour. The reaction mixture was poured into saturated ammonium chloride solution, washed with water (3 × 150 ml), and dired ($Na_2SO_4$). Rmeoval of the ether gave a pale yellow oil which solidified on standing. Recrystallisation from petroleum ether (60—80°) gave 1,1-diphenyl-2-chloro-ethan-1-ol (60%) as a white crystalline solid, mp. 56—57°.

*Stage 2.* 1,2,4-Triazole (0.03 mol, 2.07 g) was added portionwise to a suspension of sodium hydride (0.03 mol, 0.72 g) in DMF (30 ml) and the solution stirred until effervescence ceased. 1,1-Diphenyl-2-chloro-ethan-1-ol (0.015 mol, 2.94 g) in dimethylformamide (DMF; 10 ml) was added dropwise and the solution

warmed at 100° for six hours. The reaction mixture was poured into water and a white solid crystallised out. This was filtered off, washed with water, dried, and recrystallised from ethanol to give title compound as a white crystalline solid, mp. 128—129°.

Example 4

1-(1,2,4-Triazol-1-yl)-2-phenyl-4-methyl-pentan-2-ol (Compound 31)

*Stage 1.* The Grignard reagent generated from isobutyl bromide (0.1 mol, 13.7 g) in sodium diethyl ether (50 ml) and magnesium turnings (0.11 g atoms; 2.6 g) was added dropwise to a solution of phenacyl chloride (0.05 mol, 7.7 g) in sodium dry diethyl ether (100 ml) so that gentle reflux was maintained. The solution was then stirred at room temperature for 1 hour and the magnesium complex destroyed by pouring into a saturated ammonium chloride solution (200 ml). The ethereal extract was washed with water (3 × 150 ml) and dried ($Na_2SO_4$). Removal of the solvent gave a colourless liquid which distilled at reduced pressure to give 2-methyl-4-phenyl-5-chloro-pentan-4-ol (70%), bp. 86—88°/0.01 mm Hg.

*Stage 2.* 1,2,4-Triazole (0.03 mo, 2.07 g) was added portionwise to 100% sodium hydride (0.03 mol, 0.72 g) in dry DMF (30 ml) and stirred at room temperature until the effervescence ceased. 2-Methyl-4-phenyl-5-chloro-pentan-4-ol (0.01 mol, 21. g) in dry DMF (10 ml) was added dropwise at room temperature and then the solution was stirred at 100° for 6 hours. On cooling to room temperature the solution was poured into water to precipitate out a solid solution which was recrystallised from petroleum (60—80°)/chloroform giving the title compound (60%) as a white crystalline solid, mp. 90—95°.

Example 5

1-(1,2,4-Triazol-1-yl)2,o-chlorophenyl-2-p-fluorophenyl-ethan-2-ol (Compound 45)

A solution of dimethyl oxosulphonium methylide was prepared under nitrogen from sodium hydride (0.03 mol) and powdered trimethyl oxosulphonium iodide (0.03 mol) in dry dimethylsulphoxide (DMSO; 30 ml). A solution of o-chlorophenyl-p-fluorophenyl ketone (0.025 mol) in DMSO (10 ml) was added dropwise at room temperature. The solution was then heated at 50° for 1½ hours, cooled to room temperature and poured into water. The solution was extracted with diethyl ether (100 ml), washed with water (3 × 100 ml), and dried over anhydrous sodium sulphate. Removal of the solvent gave 1-o-chlorophenyl-1-p-fluorophenyl ethylene oxide (90%) as a colourless liquid.

1,2,4-Triazole (0.04 mol) was added portionwise to sodium hydride (0.04 mol) in DMF (40 ml) and the solution stirred at room temperature until effervescence ceased. 1-o-Chlorophenyl-1-p-fluorophenyl ethylene oxide (0.02 mol) in DMF (10 ml) was added dropwise and the solution stirred at 80° for 4 hours. The solution was poured into water and triturated with petroleum ether to give a white crystalline solid which was filtered off and dried. Recrystallisation from petroleum ether (60—80°)/methylene chloride gave the title compound (70%) as a white crystalline solid, mp. 115—116°.

**Claims**

1. Chemical compounds of the general formula:

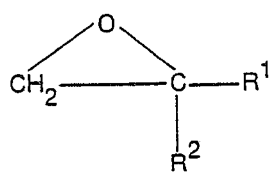 and 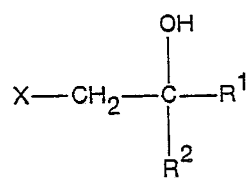

wherein X is a halogen atom, $R^1$ is butyl (especially *t*-butyl), 2-chloro- or 2-fluorophenyl, or cycloalkyl and $R^2$ is 2-chlorobenzyl but only when $R^1$ is butyl or cycloalkyl, or $R^2$ is:

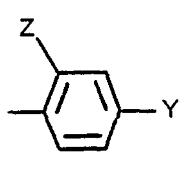

wherein Z is hydrogen or chlorine and Y is chlorine or fluorine provided that Z and Y are both chlorine only when $R^1$ is butyl.

2. Chemical compounds as claimed in claim 1 wherein $R^1$ is butyl (*n-*, *sec-*, *iso-* or *t*-butyl), 2-chloro- or 2-fluorophenyl, or cycloalkyl and $R^2$ is 2-chlorobenzyl, 4-chlorophenyl, 4-fluorophenyl or 2-chloro-4-fluorophenyl or 2,4-dichlorophenyl only when $R^1$ is butyl.

3. Chemical compounds as claimed in claim 1 or claim 2 wherein $R^1$ is *t*-butyl, 2-chlorophenyl or 2-fluorophenyl and $R^2$ is 4-fluorophenyl or 4-chlorophenyl.

4. The compounds of formula:

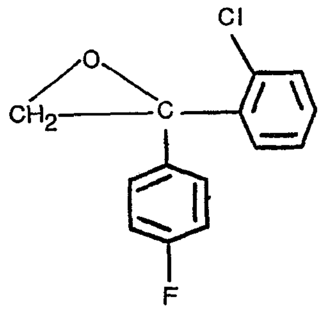 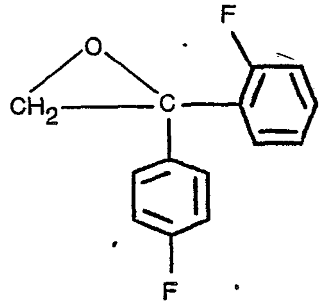 and 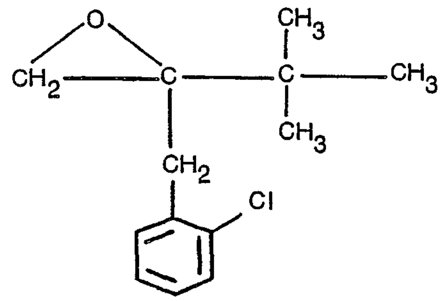

5. The compounds having the specific formulae below wherein X is chlorine or bromine:

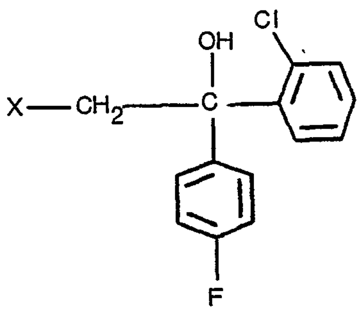 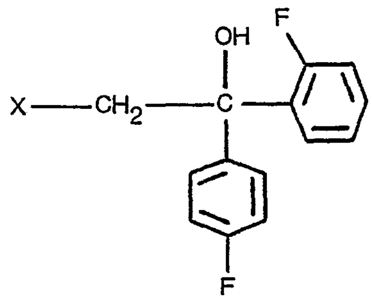 and 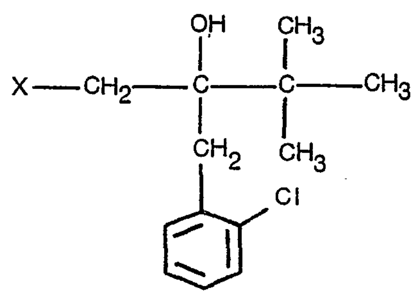

**Patentansprüche**

1. Chemische Verbindungen der allgemeinen Formeln

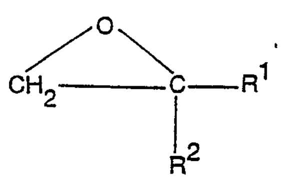 und 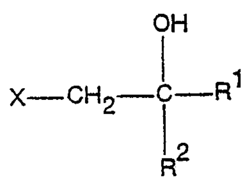

worin X für ein Halogenatom steht, $R^1$ für Butyl (insbesondere t-Butyl), 2-Chloro- oder 2-Fluorophenyl oder Cycloalkyl steht und $R^2$ für 2-Chlorobenzyl steht, aber nur, wenn $R^1$ Butyl oder Cycloalkyl bedeutet, oder $R^2$ für

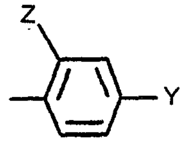

steht, worin Z Wasserstoff oder Chlor bedeutet und Y Chlor oder Fluor bedeutet, mit der Maßgabe, daß Z und Y nur dann beide Chlor sind, wenn $R^1$ Butyl bedeutet.

2. Chemische Verbindungen nach Anspruch 1, worin $R^1$ für Butyl (n-, sec-, iso- oder t-Butyl), 2-Chloro- oder 2-Fluorophenyl oder Cycloalkyl steht und $R^2$ für 2-Chlorobenzyl, 4-Chlorophenyl, 4-Fluorophenyl oder 2-Chloro-4-fluorophenyl steht oder, nur wenn $R^1$ Butyl bedeutet, für 2,4-Dichlorophenyl steht.

3. Chemische Verbindungen nach Anspruch 1 oder 2, worin $R^1$ für t-Butyl, 2-Chlorophenyl oder 2-Fluorophenyl steht und $R^2$ für 4-Fluorophenyl oder 4-Chlorophenyl steht.

4. Die Verbindungen der Formel

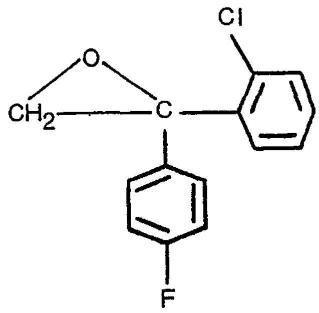
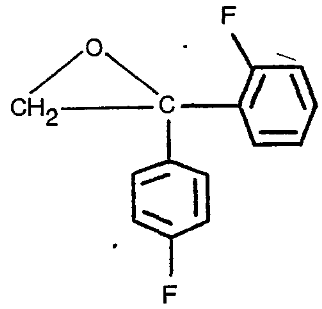
und
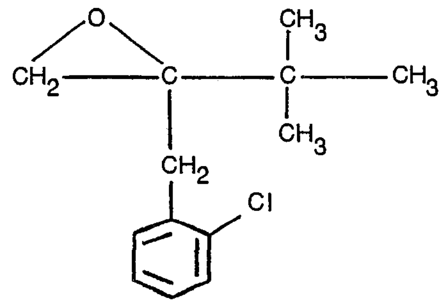

5. Verbindungen, welche die unten angegebenen speziellen Formeln aufweisen, worin X für Chlor oder Brom steht:

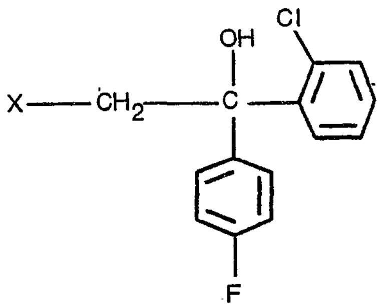
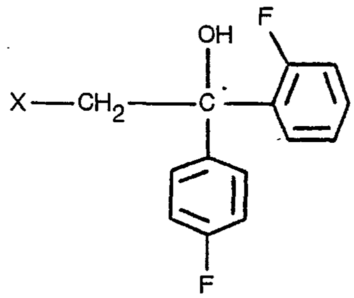
und
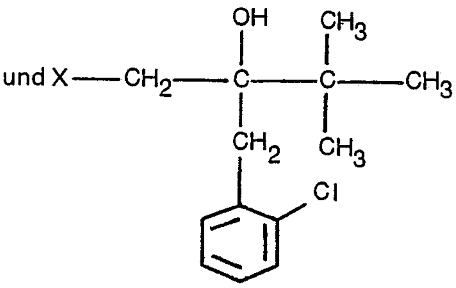

**Revendications**

1. Composés chimiques de formule générale

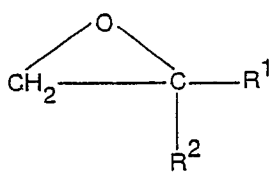
et
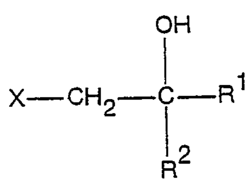

où X est un atome d'halogène, $R^1$ est un groupe butyle (notamment tertio-butyle), 2-chloro- ou 2-fluorophényle ou un groupe cycloalkyle et $R^2$ est un groupe 2-chlorobenzyle, mais uniquement lorsque $R^1$ est un groupe butyle ou cycloalkyle, ou bein $R^2$ répond à la formule:

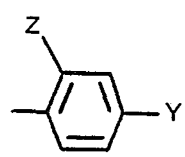

dans laquelle Z est l'hydrogène ou le chlore et Y est le chlore ou le fluor, sous réserve que Z et Y ne soient tous les deux du chlore que lorsque $R^1$ est un groupe butyle.

2. Composés chimiques suivant la revendication 1, dans lesquels $R^1$ est un groupe butyle (n-, sec.- iso- ou tertio-butyle), 2-chloro- ou 2-fluorophényle, ou cycloalkyle et $R^2$ est un groupe 2-chlorobenzyle, 4-chlorophényle, 4-fluorophényle ou 2-chloro-4-fluorophényle, ou un groupe 2,4-dichlorophényle seulement lorsque $R^1$ est un groupe butyle.

3. Composés chimiques suivant la revendication 1 ou la revendication 2, dans lesquels $R^1$ est un groupe tertio-butyle, 2-chlorophényle our 2-fluorophényle, et $R^2$ est un groupe 4-fluorophényle ou 4-chlorophényle.

4. Composés de formule:

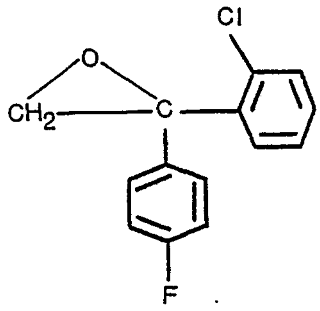 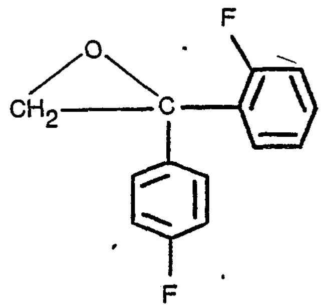 et 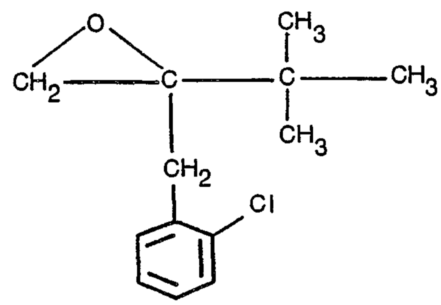

5. Composés répondant aux formules particulières ci-dessous dans lesquelles X est le chlore ou le brome:

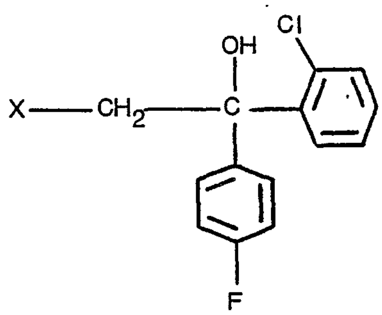 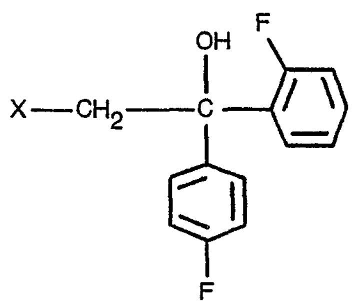 et